(19)

**Europäisches Patentamt**
**European Patent Office**
**Office européen des brevets**

(11) **EP 4 278 951 A1**

(12) **EUROPEAN PATENT APPLICATION**

(43) Date of publication:
**22.11.2023 Bulletin 2023/47**

(21) Application number: **22173473.4**

(22) Date of filing: **16.05.2022**

(51) International Patent Classification (IPC):
**A61B 5/00** *(2006.01)* **A61B 5/1455** *(2006.01)*
**A61B 5/113** *(2006.01)*

(52) Cooperative Patent Classification (CPC):
**A61B 5/0075; A61B 5/113; A61B 5/14552;**
**A61B 5/6823; A61B 5/6833; A61B 5/721;**
**A61B 5/746**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **CARAG AG**
**6340 Baar (CH)**

(72) Inventors:
- **NAPOLETANO, Daniel**
  **8455 Rüdlingen (CH)**
- **ISLER, Helene**
  **8051 Zürich (CH)**
- **BERNHARD, Jérôme**
  **8003 Zürich (CH)**

(74) Representative: **Grünecker Patent- und**
**Rechtsanwälte**
**PartG mbB**
**Leopoldstraße 4**
**80802 München (DE)**

(54) **SPECTROPHOTOMETRIC DEVICE FOR MEASURING BLOOD OXYGEN SATURATION**

(57)     According to a first aspect, the invention relates to a spectrophotometric device for measuring blood oxygen saturation in a subject's tissue comprising one or more light sources for emitting a light signal into the subject's tissue, one or more detectors for detecting the light signal reflected by the subject's tissue, an electronic data processing unit configured to calculate the blood oxygen saturation based on the light signal measured by the one or more detectors, and a motion sensor for detecting a motion signal indicative of motion of the light sources and/or the light detectors. The device is characterized in that the electronic data processing unit is configured to discard the light signal measured by the one or more detectors, if the motion signal detected by the motion sensor exceeds a predetermined threshold value. Further, the invention relates to a spectrophotometric device for measuring blood oxygen saturation in a subject's tissue comprising at least one light source for emitting a light signal into the subject's tissue, at least one detector for detecting the light signal reflected by the subject's tissue, an electronic data processing unit configured to calculate the blood oxygen saturation taking into account the light signal measured by the at least one detector, and a contact sensor for detecting a contact signal indicative of contact between the subject's skin and the light source and/or the detector.

EP 4 278 951 A1

**Description**

The present invention relates to a spectrophotometric device for measuring blood oxygen saturation in a subject's tissue as well as a method for measuring blood oxygen saturation in a subject's tissue.

**Technical Background**

Monitoring blood oxygen saturation in a subject's tissue is of clinical importance, since low blood oxygen saturation is indicative of potentially lethal disorders. This is, for example, the case for preterm infants, which often suffer from impairments of the gestational tract such as necrotizing enterocolitis or obstipation, and are at a constant risk of developing shock. In the case of preterm infants, there is, therefore, the need to constantly and accurately monitor the abdominal oxygen saturation.

The blood oxygen saturation in a subject's tissue is defined as:

$$StO_2 = c(HbO_2) / (c(HbO_2) + c(Hb))$$

where $c(HbO_2)$ and $c(Hb)$ are the concentrations of oxyhemoglobin and deoxyhemoglobin, respectively.

Near-infrared spectroscopy (NIRS) is a non-invasive technique to measure blood oxygen saturation in a subject's tissue. NIRS relies on the distinct absorption characteristics of oxyhemoglobin ($HbO_2$) and deoxyhemoglobin (Hb) in the near-infrared spectral range in order to determine the relative concentrations of $HbO_2$ and Hb. NIRS can be performed non-invasively by placing a spectroscopic sensor on a subject's skin and measuring the attenuation of a light signal after it has passed through the subject's tissue.

The measured light attenuation is related to the concentration of a given light absorbing species (chromophore) by the Lambert-Beer law:

$$A_\lambda = - \log (I_\lambda/I_{\lambda 0}) = \varepsilon_\lambda \, c \, d$$

where $A_\lambda$ is the light attenuation at a particular wavelength A, c is the concentration of a particular chromophore, $\varepsilon_\lambda$ is the extinction coefficient of a particular chromophore at a particular wavelength, and d the light source to detector separation distance. Using the known extinction coefficient, a chromophore's concentration can be calculated from the measured light attenuation. In the case of a mixture of different chromophores, the relative concentrations of the chromophores can be determined by measuring light attenuation at several distinct wavelengths, at which the extinction coefficients of the chromophores differ. For a mixture comprising N different chromophores, this requires measuring the attenuation at a minimum of N different wavelengths.

In a typical NIRS apparatus, a light signal of a known wavelength and intensity is transmitted into a subject's tissue and the light that is transmitted through or diffusely reflected from the tissue is detected to calculate the light attenuation. To accurately determine the concentration of chromophores in a tissue from the measured light attenuation, it is necessary to account for the optical properties of the tissue, in particular absorption due to other chromophores present in the tissue and the tissue's scattering properties. In practice, the tissue's scattering properties need to be accounted for by calibration measurements. To account for chromophores other than $HbO_2$ and Hb, the absorption spectra of these chromophores have to be determined in order to estimate the wavelength-dependent extinction coefficients, and the light attenuation has to be measured at a minimum of 2+M wavelengths, where M is the number of additional chromophores that should be accounted for. Several methods for addressing these problems have been developed in the prior art.

EP 1 259 791 B1 discloses a NIRS method for measuring the total blood oxygen saturation within a subject's tissue by measuring the light attenuation at three or more wavelengths and calculating the difference in attenuation between the wavelengths. This approach is also known as the "differential wavelength method". This method requires measuring at N+1 different wavelengths in order to determine the concentrations of N different chromophores. By determining the differential attenuation, the contributions of tissue light scattering, fixed light absorbing components, and measuring apparatus characteristics are minimized relative to the attenuation attributable to $HbO_2$ and Hb, which improves the accuracy of the measured blood oxygen saturation.

US 2012/0136225 A1 discloses a method for determining the blood oxygen saturation within a subject's lower gastro-intestinal tissue that involves taking into account the presence of wavelength dependent absorbing material not present in blood. Specifically, US 2012/0136225 A1 suggests taking into account light attenuation due to stool present in a subject's lower gastro-intestinal tract, particularly meconium present in the gastro-intestinal tract of new-born infants. US 2012/0136225 A1 also teaches the use of the differential wavelength method for analyzing NIRS data.

Although the differential wavelength method minimizes the contribution of the scattering properties of the tissue, it still requires calibration to account for scattering as well as unspecific background absorption. This calibration is performed by determining the blood oxygen saturation of a given reference tissue assuming that the oxygen saturation of said reference tissue is the weighted sum of the oxygen saturation of a subject's venous and arterial blood. This, however, requires knowledge of the relative contributions of venous and arterial blood in that tissue. Although empirical data for the relative contributions of venous and arterial blood oxygen saturation exist, the reliability of this data is questionable. Thus, the available calibration methods present a potential source of error for the differential wavelength method.

An alternative method for performing NIRS measurements is to measure light attenuation at several wavelengths and at several different distances between the light source and the light detectors. The absorption $\mu_{a,\lambda}$ at a particular wavelength A can then be calculated based on the following equation:

$$\mu_{a,\lambda} = 1/(3 \; \mu_{s,\lambda}) \; (\ln 10 \; \partial A_{\lambda}/\partial d - 1/d)^2$$

where $\mu_{s,\lambda}$ is an empirically determined value that accounts for attenuation of the light signal due to light scattering in the subject's tissue at the particular wavelength A, $A_{\lambda}$ is the attenuation at the particular wavelength A, d is the mean distance between light source and detectors, and $\partial A_{\lambda}/\partial d$ is the slope of the attenuation versus the light source to detector distance. The concentration of chromophores can be calculated from the absorption $\mu_{a,\lambda}$ using the Lambert-Beer law. This approach is also known as the "multi-distance method". It has been applied to measure the blood oxygen saturation of muscle tissue (Tachtsidis, Ilias et al. "A Hybrid Multi-Distance Phase and Broadband Spatially Resolved Spectrometer and Algorithm for Resolving Absolute Concentrations of Chromophores in the Near-Infrared Light Spectrum." Advances in Experimental Medicine and Biology 662 (2010): 169-175).

Conventional spectrophotometric devices for measuring blood oxygen saturation can be attached to a subject's finger or can be placed flat on a subject's skin. For measuring the blood oxygenation of newborn or preterm infants, it is important to attach the devices as loosely as possible to prevent any discomfort or injury to the infant. Therefore, clamp-on devices that are attached to a finger are less suitable for monitoring newborn or preterm infants. Instead, spectrophotometric devices that are more or less loosely placed on a subjects abdomen are more suitable for monitoring newborn or preterm infants. However, the device must be attached tightly enough to prevent the device from falling off and to prevent interference from ambient light. Because, they cannot be attached too tightly, conventional spectrophotometric devices for measuring blood oxygen saturation sometimes suffer from measurement artifacts caused, for example, by movement of the subject or a loss of skin contact with the subject.

**Technical problem**

It is an object of the present invention to provide an improved a spectrophotometric device for measuring blood oxygen saturation in a subject's tissue, in particular for measuring blood oxygen saturation in newborn or preterm infants. The spectrophotometric device should have improved measurement accuracy and/or should provide information on other vital parameters in addition to blood oxygen saturation.

**Description of the invention**

According to a first aspect, the invention relates to a spectrophotometric device for measuring blood oxygen saturation in a subject's tissue comprising one or more light sources for emitting a light signal into the subject's tissue, one or more detectors for detecting the light signal reflected by the subject's tissue, an electronic data processing unit configured to calculate the blood oxygen saturation based on the light signal measured by the one or more detectors, and a motion sensor for detecting a motion signal indicative of motion of the light sources and/or the light detectors. The device is characterized in that the electronic data processing unit is configured to discard the light signal measured by the one or more detectors, if the motion signal detected by the motion sensor exceeds a predetermined threshold value.

According to the first aspect, the invention further relates to a method for non-invasively measuring blood oxygen saturation in a subject's tissue, comprising the steps of: using at least one light source to emit a light signal into the subject's tissue, using at least one detector to detect the light signal reflected from the subject's tissue, using an electronic data processing unit to calculate the blood oxygen saturation based on the light signal measured by the one or more detector, using a motion sensor to detect a motion signal indicative of motion of the light source and/or the light detector. The method is characterized in that the electronic data processing unit discards the light signal measured by the one or more detectors, if the motion signal detected by the motion sensor exceeds a predetermined threshold value.

By detecting and taking into account a motion signal indicative of motion of the light source and/or the light detector, the spectrophotometric device can eliminate one source of measurement artifacts and thereby improve the accuracy of the measured blood oxygen saturation. This is particularly important when monitoring newborn or preterm infants, who often display sudden movement leading to measurement artifacts in conventional spectrophotometric devices.

In the context of the present invention, discarding the light signal means that the normal processing of the light signal is interrupted. This may mean, for example, that no blood oxygen saturation is calculated, i.e. that the calculation of the blood oxygen saturation is interrupted, that the blood oxygen saturation is calculated but not reported to the user or that the blood oxygen saturation is calculated and reported but is labelled as an uncertain value. If the blood oxygen saturation is calculated under these conditions, it may be saved in an electronic record for later use, but it will preferably be labelled as an uncertain value.

The spectrophotometric device is configured to transmit a light signal into a subject's tissue and to detect the light signal after it has passed through the subject's tissue. Thereby, the spectrophotometric device may measure the attenuation of the light signal after it has passed through the subject's tissue. The light signal transmitted into the subject's tissue is partly absorbed, partly transmitted and partly reflected from the subject's tissue. The spectrophotometric device according to the invention measures the light signal that is reflected from within the subject's tissue. Therefore, the at least one light source and the at least one detector are preferably positioned on one the same side relative to the subject's tissue.

Thereby, the spectrophotometric device differs from conventional clamp-on devices where the light source and the detector are positioned on opposite sides of the subject's tissue in order to measure the light signal transmitted through the subject's tissue. Such conventional clamp-on devices can only be attached to the extremities of a subject, e.g. a subject's finger, and thus cannot be used to measure blood oxygen saturation on other body parts, such as the abdomen. In contrast thereto, the spectrophotometric device according to the present disclosure can be placed on any body part, e.g. on a subject's abdomen, to measure the blood oxygen saturation specifically in that body part.

Preferably, the spectrophotometric device is configured to measure blood oxygen saturation on the subject's head, torso, abdomen, arm or leg, more preferably the subject's head, torso or abdomen. Most preferably, the spectrophotometric device is configured to measure blood oxygen saturation on a subject's abdomen. This requires the spectrophotometric device to be configured so that the light sources and the light detectors can be placed flat on the subject's skin. Preferably, the spectrophotometric device also comprises a fixing means, with which the light sources and the light detectors can be immobilized on the subject's skin. This can be, for example, a belt or an adhesive patch. In a preferred embodiment, the spectrophotometric device comprises an adhesive patch, preferably a Velcro patch or hook-and-loop fastener, which can be used to attach the spectrophotometric device to the subject's clothing, for example to attach the spectrophotometric device underneath a subject's diaper.

The light source may be a broadband light source emitting light over a range of wavelengths. Alternatively, the light source may be a collection of light sources each emitting light at a narrow spectral bandwidth, such as a collection of light emitting diodes. In a preferred embodiment, the light source includes a collection of light emitting diodes each emitting light at a different wavelength.

The spectrophotometric device may comprise one or more such light sources, each being either a broadband light source or a collection of light sources emitting light in a narrow spectral bandwidth.

The light detector may be, for example, a CCD sensor or a photodiode or any other device that can convert light to an electrical current or voltage. The detector may comprise a collection of individual detectors, each of which is configured to detect light at a different wavelength. For example, the detector may comprise an array of photodiodes, each equipped with a filter which transmits light of a certain wavelength onto the respective photodiode. Alternatively, the detector may comprise an array of photodiodes or a CCD sensor and a diffraction grating which splits the light signal into different light components having distinct wavelengths and images each light component onto a distinct photodiode of the array of photodiodes or onto a distinct area of the CCD sensor. As yet another alternative, the spectrophotometric device may employ time or frequency multiplexing for wavelength separation. Thereby, the detector is able to measure the intensity of the light signal as a function of wavelength. This enables the spectrophotometric device to measure the attenuation of the light signal as a function of the wavelength.

The spectrophotometric device may comprise one or more such light detectors, where each light detector may comprise, for example, an array of photodiodes, a single CCD chip or a single photodiode. Preferably, the spectrophotometric device comprises two to six, most preferably four such light detectors positioned at different distances to the light source.

Preferably, the spectrophotometric device is configured to measure the attenuation of the reflected light signal at two or more distinct wavelengths. Measuring the attenuation at two or more distinct wavelengths allows to calculate the relative concentrations of oxyhemoglobin and deoxyhemoglobin in the subject's tissue and thereby calculating the blood oxygen saturation.

In order to measure the attenuation at a given number of distinct wavelengths, it is sufficient that the light source and the light detector are configured to measure the attenuation at distinct wavelength ranges, which at least include the specified wavelength. The spectral bandwidth of each wavelength range may vary, as long as the wavelength ranges can be clearly distinguished. Preferably, the attenuation is measured at distinct wavelength ranges having a bandwidth of ± 25 nm or less, more preferably ± 15 nm or less, even more preferably ± 10 nm or less, most preferably ± 5 nm or less.

In order to increase the accuracy of the measurement, attenuation is preferably measured at three or more distinct wavelengths, more preferably four or more distinct wavelengths, more preferably at five or more, most preferably at seven or more. Measuring at three or more distinct wavelengths enables the use of the differential wavelength method for calculating the relative concentrations of oxyhemoglobin and deoxyhemoglobin.

To distinguish between deoxyhemoglobin and oxyhemoglobin, it is preferable to measure at least at two different wavelengths where the extinction coefficients of both species differ strongly, i.e to measure at least at one wavelength where deoxyhemoglobin has a higher extinction coefficient than oxyhemoglobin and at least at a second wavelength where oxyhemoglobin has a higher extinction coefficient than deoxyhemoglobin.

Preferably, the spectrophotometric device is configured to measure the attenuation of the reflected light signal at distinct wavelengths in the range of 650 nm to 3 $\mu$m, more preferably in the range of 650 nm to 1 $\mu$m, most preferably in the range of 690 nm to 910 nm.

In the case of performing the measurement on a subject's abdomen, several combinations of wavelengths have been found that offer an increased measurement accuracy. These wavelengths can be selected to better distinguish between Hb, $HbO_2$, and other absorbers present in a subject's abdomen, such as stool. These optimized combinations of wavelengths are set out in the following.

In one embodiment, the spectrophotometric device is configured to measure the attenuation of the light signal at distinct wavelengths selected from 695 nm, 712 nm, 733 nm, 743 nm, 762 nm, 783 nm, 790 nm, 805 nm, 880 nm, 894 nm, and 909 nm. Preferably, the wavelengths are selected from 695 nm, 733 nm, 762 nm, 790 nm, 805 nm, 894 nm, and 909 nm. Preferably, these wavelengths are measured with a spectral bandwidth of ± 10 nm, more preferably ± 5 nm. Preferably, the spectrophotometric device is configured to measure the attenuation of the light signal at least at three distinct wavelengths selected from these wavelengths.

In one embodiment, the spectrophotometric device is configured to measure of the attenuation of the light signal at least at 712 nm, 733 nm, 762 nm, 783 nm, and 909 nm. Preferably, these wavelengths are measured with a spectral bandwidth of ± 10 nm, more preferably ± 5 nm.

In another embodiment, the spectrophotometric device is configured to measure of the attenuation of the light signal at least at 712 nm, 733 nm, 762 nm, 783 nm, 894 nm, and 909 nm. Preferably, these wavelengths are measured with a spectral bandwidth of ± 10 nm, more preferably ± 5 nm.

Most preferably, the spectrophotometric device is configured to measure the attenuation of the light signal at 695 nm, 733 nm, 762 nm, 790 nm, 805 nm, 894 nm, and 909 nm. Preferably, these wavelengths are measured with a spectral bandwidth of ± 10 nm, more preferably ± 5 nm.

In a preferred embodiment, the spectrophotometric device is configured to measure of the attenuation of the light signal at two or more light source to detector distances. This enables the use of the multi-distance method for calculating the relative concentrations of oxyhemoglobin and deoxyhemoglobin.

To implement this feature, the spectrophotometric device preferably comprises at least two light detectors positioned at different, fixed distances from the light source. In a preferred embodiment, the spectrophotometric device comprises one light source at a first position and two or more light detectors positioned at different distances from the light source. However, it is likewise possible that the spectrophotometric device comprises at least two light sources positioned at different, fixed distances from a light detector. Further, it is possible that the spectrophotometric device comprises a single light source and a single light detector, wherein the light source and/or the light detector are movable in order to vary the light source to detector distance during the measurement. This embodiment has the advantage that the attenuation of the light signal as a function of the light source to detector distance can be sampled over a wide range and a large number of data points.

In a preferred embodiment, the spectrophotometric device is configured to measure the attenuation at more than two light source to detector distances in order to improve the accuracy of the multi-distance method. In a preferred embodiment, the spectrophotometric device is configured to measure the attenuation at three light source to detector distances.

The distance between the light source and the light detector may be optimized based on, for example, the sensitivity of the detectors and the optical properties of the subject's tissue. In the case of a spectrophotometric device for measuring the blood oxygen saturation in the abdomen of a new-born infant, the shortest light source to detector distance is preferably at least 0.8 cm, more preferably at least 0.9 cm, and most preferably at least 1.0 cm. Preferably, the shortest distance between the light source and the detectors is in the range of 0.8 to 2 cm, more preferably at least 0.9 to 1.5 cm, and most preferably 0.95 to 1.2 cm. The longest light source to detector distance is preferably in the range of 2 to 10 cm, preferably 2.2 to 6 cm, most preferably 2.5 to 5 cm.

Preferably, the at least one light source and the at least one light detector are configured such that they can be brought into direct contact with the subject's skin in order to avoid any interference with ambient light.

The one or more light sources and the one or more light detectors are preferably positioned in a single housing. The housing is preferably configured to be attached to or positioned on the subject's skin. More specifically, the housing is preferably configured to be attached to or positioned on the subject's abdomen.

The housing may be made from any suitable material, preferably from a plastic material. The housing has at least one surface facing the subject's skin, and the one or more light sources and the one or more light detectors are positioned on this surface. Preferably, the housing has a single surface facing the subject's skin and both the light sources and the light detectors are positioned on this surface.

The surface facing the subject's skin may be planar or it may be shaped to correspond to the surface of the body part, on which the spectrophotometric device is placed. For example, the surface of the housing may be concavely curved to correspond to the convex curvature of a subject's abdomen.

In a preferred embodiment, the housing is provided with an adhesive patch for attaching the housing to the subject's clothing. The adhesive patch may preferably be provided on a surface of the housing facing away from the subject's skin, preferably on a surface opposite the abovementioned surface on which the light sources and light detectors are provided. For example, the adhesive patch may be used to attach the housing to a diaper worn by the subject. Thereby, the spectrophotometric device can be fixed underneath the subject's clothing, such as a diaper. The adhesive patch is preferably a Velcro patch or hook-and-loop fastener.

Preferably, the housing is made from a rigid material to that the distance between the light source and the detector remains constant. This is particularly preferable, if the spectrophotometric device uses the multi-distance method for calculating the blood oxygen saturation. Alternatively, the housing may be made from a flexible material. This has the advantage that the shape of the housing can be adapted to the individual subject and the shape of the individual body part. This allows a better fit between the spectrophotometric device and the subject's skin and may help to eliminate measurement artifacts arising from ambient light hitting the light detector.

According to the first aspect, the spectrophotometric device further comprises a motion sensor. The motion sensor generates a motion signal indicative of motion of the light source and/or the light detector. Therefore, the motion sensor is preferably positioned in close proximity to the light source and/or light detector. More preferably, the motion sensor is provided in the above-described housing together with the one or more light sources and the one or more light detectors. In this case, the motion signal is preferably indicative of movement of the entire housing. However, it is also possible that the motion signal is indicative specifically for motion of one of the light sources and/or one of the light detectors.

The motion sensor may comprise, for example, a linear accelerometer configured to measure linear displacement or a gyroscope configured to measure rotational displacement. A combined linear accelerometer and gyroscope may also be used to measure both linear displacement and rotational displacement. Preferably, the motion sensor is piezoelectric device or a microelectromechanical system (MEMS).

Preferably, a linear accelerometer is used. A suitable linear accelerometer may measure displacement along three axes, two axes or a single axis. Most preferably, a linear 3-axis accelerometer is used.

The motion sensor preferably has a sampling rate of 1 kHz to 5.3 kHz. The motion sensor preferably measures acceleration in the range of $\pm$ 16 g, more preferably $\pm$ 8 g, most preferably $\pm$ 4 g, more preferably $\pm$ 2 g.

The electronic data processing unit configured to calculate the blood oxygen saturation is configured to receive data from both the motion sensor and the one or more light sources and one or more light detectors. The electronic data processing unit may be physically connected to the motion sensor, the light sources and light detectors, e.g. by an electrical wire connection. However, the electronic data processing unit may also be positioned at a remote location and be connected wirelessly to the motion sensor, the light sources and light detectors. The electronic data processing unit may also be a central processing unit that is part of a multitude of spectrophotometric devices. In this case, the electronic data processing unit receives signals of a multitude of motion sensors, light sources and light detectors to calculate blood oxygen saturation for a multitude of subjects.

The electronic data processing unit may be implemented, for example, on a personal computer comprising a central processing unit, a random access memory, a data storage device and a data connection for receiving the measurement data from the motion sensor and the light detectors.

The electronic data processing unit carries out an algorithm for calculating the blood oxygen saturation. In this context, blood oxygen saturation $StO_2$ is defined as the ratio of the concentration of oxyhemoglobin $HbO_2$ to the total concentration of oxyhemoglobin $HbO_2$ to deoxyhemoglobin Hb according to the following equation:

$$StO_2 = c(HbO_2)/(c(HbO_2) + c(Hb))$$

where $c(HbO_2)$ is the concentration of oxyhemoglobin and $c(Hb)$ is the concentration of deoxyhemoglobin.

The algorithm involves determining the light attenuation $A_\lambda$ of the light signal emitted by the one or more light sources and detected by the one or more light detectors as a function of the wavelength A. From the light attenuation $A_\lambda$, the algorithm then calculates the concentrations of oxyhemoglobin $c(HbO_2)$ and the concentration of deoxyhemoglobin $c(Hb)$ based on the Lambert-Beer Law. Specifically, the algorithm uses empirically determined extinction coefficients of oxyhemoglobin and deoxyhemoglobin and empirically determined calibration data accounting for light scattering in the subject's tissue. The extinction coefficients of oxyhemoglobin and deoxyhemoglobin can be determined from absorption spectra of oxyhemoglobin and deoxyhemoglobin in isolation. Preferably, the algorithm only determines relative concentrations of oxyhemoglobin and deoxyhemoglobin. As the blood oxygen saturation is defined as the ratio of the concentrations of oxyhemoglobin and deoxyhemoglobin, it is not necessary to determine the absolute concentrations.

In one embodiment, the algorithm involves calculating the concentrations of oxyhemoglobin $c(HbO_2)$ and the concentration of deoxyhemoglobin $c(Hb)$ using the differential wavelength method. This involves measuring the light attenuation at least at three distinct wavelengths and calculating the difference in attenuation between the wavelengths. The differential wavelength method is described, for example, in EP 1 259 791 B1.

In another embodiment, the algorithm involves calculating the concentrations of oxyhemoglobin $c(HbO_2)$ and the concentration of deoxyhemoglobin $c(Hb)$ using the multi-distance method. This involves measuring the light attenuation at two or more different light source to detector distances, calculating the slope of the attenuation of the light signal versus the light source to detector distance as a function of the wavelength and calculating the blood oxygen saturation within the subject's tissue on the basis of said slope of the attenuation of the light signal. The multi-distance method is described, for example, in Tachtsidis, Ilias et al. "A Hybrid Multi-Distance Phase and Broadband Spatially Resolved Spectrometer and Algorithm for Resolving Absolute Concentrations of Chromophores in the Near-Infrared Light Spectrum." Advances in Experimental Medicine and Biology 662 (2010): 169-175.

By measuring the attenuation as a function of the light source to detector distance, it is possible to calculate the relative absorption $\mu_{a,\lambda}$ using the multi-distance approach. This removes the necessity of performing calibration by determining the blood oxygen saturation of a given reference tissue assuming that oxygen saturation of said reference tissue is the weighted sum of the oxygen saturation of a subject's venous and arterial blood. This method only needs to account for light scattering of the tissue. This removes a source of systematic error, since it no longer requires any assumptions on the relative contributions of venous and arterial blood.

The electronic data processing unit may further be configured to determine the subject's pulse and/or breathing frequency using the pulsatile part of the measured light attenuation. In this case, it is necessary that the electronic data processing unit continuously samples the light signal detected by the light detector over a given period of time.

In a preferred embodiment, the algorithm takes into account absorption from other chromophores in addition to oxyhemoglobin and deoxyhemoglobin. The algorithm preferably takes into account empirically determined data that account for wavelength dependent light absorption by light absorbers other than hemoglobin. Preferably, the algorithm takes into account light absorption due to other light absorbing species in a subject's abdomen. Most preferably, this includes absorption by stool, transitional stool of infants, meconium, and/or billiverdin.

In one particular embodiment, the algorithm takes into account the wavelength dependent extinction coefficients of these other light absorbers. The extinction coefficients can be determined by measuring the absorption spectra of, for example, isolated samples of stool, transitional stool, meconium, and/or biliverdin. In a preferred example, the extinction coefficients are determined by measuring the absorption spectra of isolated samples of meconium.

Meconium is the earliest stool of a mammalian infant. Meconium is composed of materials ingested during the time the infant spends in the uterus: intestinal epithelial cells, lanugo, mucus, amniotic fluid, bile, and water. It has been found that averaged absorption spectra of meconium samples taken from number of different subjects can be used as a source of extinction data for the above calculation. In one embodiment, the data accounting for attenuation of the light signal due to light absorbers therefore include the wavelength-dependent extinction coefficients of meconium samples taken from new-born infants.

Transitional stool is produced by a new-born infant during its first days of life. Transitional stool differs from meconium in its composition and comprises high amounts of biliverdin. Therefore, the data accounting for attenuation of the light signal due to light absorbers preferably include the wavelength-dependent extinction coefficients of transitional stool samples taken from new-born infants, preferably during the first two weeks after birth, more preferably during the first week after birth, most preferably during the first five days after birth.

In another preferred embodiment, the data accounting for attenuation of the light signal due to light absorbers therefore include the wavelength-dependent extinction coefficients of biliverdin.

The electronic data processing unit is preferably configured to continuously sample the measurement data of the one or more light detectors and to calculate the blood oxygen saturation. The sampling rate preferably is in the range of 0.1 kHz to 10 kHz, more preferably 0.5 kHz to 3 kHz, most preferably 0.6 kHz to 2 kHz. The electronic data processing unit may also perform some form of downsampling, for example by calculating an average light signal from a group of consecutive, discrete measurements provided by the one or more light detectors. Preferably, the electronic data processing unit calculates a moving average of the measurement data of the one or more light detectors and calculates the light attenuation and the blood oxygen saturation based on this moving average.

Preferably, the spectrophotometric device comprises an electronic storage device connected to the electronic data processing unit, which stores data identifying the spectrophotometric device and calibration data specific for the spectrophotometric device. During operation, the electronic data processing unit can use this information to calculate the blood oxygen saturation. Preferably, the electronic storage device is an integral part of the electronic data processing unit. However, this is not essential. The electronic storage device does not necessarily have to be positioned at the same location or in proximity of the electronic data processing unit. The electronic storage device may also be provided in the above-mentioned housing containing the light sources and detectors, or it may be positioned in a connector connecting the housing to the electronic data processing unit. The electronic storage device may further be positioned at a remote location and may be connected to the electronic data processing unit, for example, through a wireless location.

According to the first aspect of the invention, the electronic data processing unit is configured to discard the light signal measured by the at least one detector, if the motion signal detected by the motion sensor exceeds a predetermined threshold value. This improves the accuracy of the measurement and reduces false alarms, as measurement artifacts caused by movement of the spectrophotometric device and/or the subject can be identified and discarded.

The electronic data processing unit is preferably configured to continuously sample the motion signal provided by the motion sensor. This preferably occurs at the same sampling rate as the sampling of the measurement of the one or more light detectors. The electronic data processing unit may perform downsampling of the measurement data received from the motion sensor as described for the light signal received by the light detector above, e.g. by calculating a moving average of the motion signal.

Preferably, the electronic data processing unit is configured to continuously sample the measurement data of the one or more light detectors to calculate the blood oxygen saturation and to continuously sample the motion signal provided by the motion sensor. If the motion signal exceeds the predetermined threshold in this embodiment, calculation of the blood oxygen saturation is interrupted.

In one embodiment, the electronic data processing unit discards the light signal, as soon as the motion signal exceeds a predetermined first threshold value, and resumes the processing of the light signal, as soon as the motion signal falls below a predetermined second threshold value. The first threshold value and the second threshold value need not necessarily be the same. Instead, the first threshold value can be greater than or less than the second threshold value. In one embodiment, the first threshold value is greater than the second threshold value. In this case, the light signal is only discarded, if the motion signal is relatively high. Thereby, the processing of the light signal is not unnecessarily interrupted by small fluctuations of the motion signal. At the same time, the processing of the light signal is only resumed, if the motion signal falls below a relatively low second threshold value. Thereby, the processing is only resumed, if an interference of the motion with the light measurement can be safely excluded.

In one embodiment, the electronic data processing unit is configured to issue a warning to the user, if the motion signal exceeds the predetermined threshold value. This may be an optical warning, such as a light indicator or a message displayed on an electronic display, and/or an acoustic warning.

In a preferred embodiment, the electronic data processing unit performs an iterative algorithm, where each iteration comprises the following steps:

1. Receive the light signal measured by the at least one detector and the motion signal detected by the motion sensor;
2. Check the quality of the light signal and the motion signal;
3. If the quality of the light signal and the motion signal is sufficient, accept the blood oxygen saturation calculation based on the light signal, optionally report the calculated blood oxygen saturation to a user, and reset a value of a signal quality timer to zero;
4. If the quality the light signal and/or the motion signal is insufficient, discard the light signal or the blood oxygen saturation calculation and increment the value of the signal quality timer; and
5. If the value of the signal quality timer exceeds a predetermined threshold, issue a warning to the user.

In this embodiment, the electronic data processing unit stores and modifies a value of a signal quality timer during each iteration of the above mentioned algorithm. This allows the electronic data processing unit to issue a warning to the user, if the signal quality timer exceeds a predetermined threshold. In other words, the user is warned, if the signal quality is insufficient for several consecutive iterations of the algorithm. A typical threshold for the signal quality timer is in the range of 1 to 10 minutes.

To check the quality of the light signal and the motion signal, the algorithm reports an insufficient quality, if the motion signal exceeds a predetermined threshold. Otherwise, it reports a sufficient quality.

Preferably, the algorithm further checks the quality of the light signal.

Preferably, the algorithm performs the following steps to check the quality of the light signal and the motion signal:

1. If the motion signal exceeds a predetermined threshold, report an insufficient quality;
2. Else, if the signal to noise ratio of the light signal is below a predetermined threshold, report an insufficient quality;
3. Else, report a sufficient quality.

Here, the noise level for calculating the signal to noise ratio is determined based on a measurement of the ambient light when all light sources of the spectrophotometric device are switched off.

More preferably, the algorithm performs the following steps to check the quality of the light signal and the motion signal:

1. If the motion signal exceeds a predetermined threshold, report an insufficient quality;
2. Else, if the signal to noise ratio of the light signal is below a predetermined threshold, report an insufficient quality;
3. Else, if the optical signal reduction as a function of the light source to detector distance does not follow an exponential law, report an insufficient signal quality;
4. Else, report a sufficient quality.

Here, the optical signal reduction as a function of the light source to detector distance is preferably evaluated by curve fitting. Preferably, the logarithm of the signal amplitude as a function of the light source to detector distance is determined, curve fitting is performed using a linear regression model and the coefficient of determination is determined. The coefficient of determination (R-squared) is determined according to known statistical methods. If the coefficient of determination is above a predetermined threshold value, preferably above a threshold value of 0.97, the optical signal reduction as a function of the light source to detector distance is considered to follow an exponential law.

If the electronic data processing unit receives a motion signal from a linear accelerometer and/or a gyroscope, the electronic data processing unit may calculate a processed motion signal indicative of motion in a particular direction. Then, this processed motion signal is compared to a predetermined threshold to decide whether or not to interrupt calculation of the blood oxygen saturation. For example, the electronic data processing unit may calculate a processed motion signal indicative of motion tangential or perpendicular to the subject's skin. This can be achieved, for example, by using a gyroscope to determine the three-dimensional orientation of the motion sensor relative to the subject's skin and by using a linear accelerometer to determine linear motion in a certain direction relative to the subject's skin. The electronic data processing unit may also calculate a processed motion signal based on a weighted average over the motion in different directions. For example, motion in a direction perpendicular to the subject's skin may be given a larger weight than motion tangential to the subject's skin. Thereby, the algorithm for calculating blood oxygen saturation is more sensitive for motion in a direction perpendicular to the subject's skin than to motion tangential to the subject's skin. A motion perpendicular to the skin may indicate that the motion sensor is detached from the skin and that the measurement of the light signal is compromised.

In a further embodiment, the electronic data processing unit is configured to determine the breathing frequency of the subject based on the motion signal provided by the motion sensor. Preferably, the electronic data processing unit is configured to determine the breathing frequency based on a periodic change in the motion signal. More preferably, this measurement is based on the motion signal provided by a linear motion sensor. More preferably, the measurement is based on a measurement of linear motion perpendicular to the subject's skin. The periodic change of the motion signal is indicative of a periodic movement of the motion sensor, which in turn is caused by movement of the subject's body, such as the subject's belly, and is thus indicative to the breathing frequency.

The spectrophotometric device may further comprise an electronic display for displaying the currently calculated blood oxygen saturation to the user.

The spectrophotometric device may further comprise a temperature sensor for measuring the skin temperature and/or the body core temperature of the subject. For example, can be a resistance temperature detector, a thermocouple or a pyrometer. To determine the body core temperature, a heat flux sensor can be used.

According to a second aspect, the invention relates to a spectrophotometric device for measuring blood oxygen saturation in a subject's tissue comprising at least one light source for emitting a light signal into the subject's tissue, at least one detector for detecting the light signal reflected by the subject's tissue, an electronic data processing unit configured to calculate the blood oxygen saturation taking into account the light signal measured by the at least one detector, and a contact sensor for detecting a contact signal indicative of contact between the subject's skin and the light source and/or the detector.

According to the second aspect, the invention further relates to a method for non-invasively measuring blood oxygen saturation in a subject's tissue, comprising the steps of: using at least one light source to emit a light signal into the subject's tissue, using at least one detector to detect the light signal reflected from the subject's tissue, using an electronic data processing unit to calculate the blood oxygen saturation based on the light signal measured by the one or more detector, using a contact sensor for detecting a contact signal indicative of contact between the subject's skin and the at least one light source and/or the at least one detector. The method is characterized in that the electronic data processing unit discards the light signal measured by the at least one detector and/or issues a warning to the user, if the contact signal indicates that at least one light source and/or at least one detector have lost contact to the subject's skin.

The spectrophotometric device according to the second aspect corresponds to the spectrophotometric device according to the first aspect disclosed above, with the modification that the spectrophotometric device according to the second aspect does not necessarily comprise the motion sensor disclosed above. Thus, the spectrophotometric device according to the second aspect can have all the optional features described for the spectrophotometric device according to the first aspect above. The spectrophotometric device according to the second aspect may also comprise the motion sensor described above. Likewise, spectrophotometric device according to the first aspect may optionally comprise the contact sensor disclosed for the second aspect.

According to the second aspect, the spectrophotometric device comprises a contact sensor for detecting a contact signal indicative of contact between the subject's skin and the light source and/or the detector. Thereby, it is possible to continuously monitor the correct placement of the light source and/or the detector. A loss of contact between the subject's skin and the light source or the detector may result in an erroneous determination of the light attenuation, for example, due to the influence of ambient light. This source of error can be excluded by continuously monitoring the contact signal measured by the contact sensor. Thus, the spectrophotometric device according to the second aspect improves the accuracy of the measured blood oxygen saturation. This is particularly important when monitoring newborn or preterm infants, who often display sudden movement leading to a loss of skin contact in conventional spectrophotometric devices.

In this context, the term "skin contact" in connection with the light source and the detector means that the light source and detector are at the optimal distance from the skin. This can mean that the surface of the light source and/or the detector is in direct physical contact with the skin or that they are placed at a certain narrow distance, which allows an optimal transmission of the light signal to and from the subject's skin.

The contact sensor may, for example, be a capacitive sensor, conductivity sensor or pressure sensor. Preferably, the contact sensor provides a distance signal indicative of the distance between the contact sensor and the subject's skin. A distance signal below a predetermined threshold indicates that the contact sensor has lost contact to the user's skin. Preferably, the contact sensor is arranged so that loss of skin contact of the contact sensor indicates that at least one light source and/or at least one detector have lost contact to the subject's skin as well.

In a preferred embodiment, the contact sensor is provided in a housing together with the light source and the detectors. The housing can be constructed as explained above with respect to the first embodiment. Preferably, the contact sensor is provided on a surface of the housing which faces the subject's skin.

Preferably, the electronic data processing unit is configured to discard the light signal measured by the at least one detector and/or to issue a warning to the user, if the contact signal indicates that at least one light source and/or at least one detector have lost contact to the subject's skin.

In one embodiment, the electronic data processing unit is configured to continuously sample the measurement data of the one or more light detectors to calculate the blood oxygen saturation and to continuously sample the contact signal provided by the contact sensor. If the contact signal indicates that at least one light source and/or at least one detector have lost contact to the subject's skin, the light signal is discarded. More preferably, the contact sensor is configured to determine which light source and/or light detector has lost contact to the subject's skin and to discard only the light signal emitted or measured by that particular light source and/or light detector.

Further, the electronic data processing unit may also configured to issue a warning to the user, if the contact signal indicates that at least one light source and/or at least one detector have lost contact to the subject's skin. This may be an optical warning, such as a light indicator or a message displayed on an electronic display, and/or an acoustic warning.

## Claims

1. A spectrophotometric device for measuring blood oxygen saturation in a subject's tissue comprising at one or more light sources for emitting a light signal into the subject's tissue, one or more detectors for detecting the light signal reflected by the subject's tissue, an electronic data processing unit configured to calculate the blood oxygen saturation based on the light signal measured by the one or more detector, and a motion sensor for detecting a motion signal indicative of motion of the light sources and/or the light detectors, **characterized in that** the electronic data processing unit is configured to discard the light signal measured by the one or more detectors, if the motion signal detected by the motion sensor exceeds a predetermined threshold value.

2. The spectrophotometric device according to claim 1, **characterized in that** the spectrophotometric device is configured to measure blood oxygen saturation on a subject's abdomen.

3. The spectrophotometric device according to any one of claims 1 or 2, **characterized in that** the spectrophotometric device is configured to measure the attenuation of the light signal at two or more wavelengths and/or at two or more light source to detector distances.

4. The spectrophotometric device according to any one of claims 1 to 3, **characterized in that** the motion sensor comprises a linear accelerometer configured to measure linear displacement and/or a gyroscope configured to measure rotational displacement.

5. The spectrophotometric device according to any one of claims 1 to 4, **characterized in that** the electronic data processing unit is configured to continuously sample the measurement data of the one or more light detectors to calculate the blood oxygen saturation and to continuously sample the motion signal provided by the motion sensor, wherein the electronic data processing unit is configured to discard the light signal if the motion signal detected by the motion sensor exceeds the predetermined threshold value.

6. The spectrophotometric device according to any one of claims 1 to 5, **characterized in that** the electronic data processing unit is configured to discard the light signal, as soon as the motion signal exceeds a predetermined first threshold value, and to resume the processing of the light signal, as soon as the motion signal falls below a predetermined second threshold value.

7. The spectrophotometric device according to any one of claims 1 to 6, **characterized in that** it further comprises an electronic storage device connected to the electronic data processing unit, which stores data identifying the spectrophotometric device and calibration data specific for the spectrophotometric device.

8. The spectrophotometric device according to any one of claims 1 to 7, **characterized in that** the electronic data processing unit is configured to determine the breathing frequency of the subject based on the motion signal provided by the motion sensor.

9. The spectrophotometric device according to any one of claims 1 to 8, **characterized in that** the electronic data processing unit is configured to issue a warning to the user, if the motion signal exceeds the predetermined threshold value.

10. The spectrophotometric device according to any one of claims 1 to 9, **characterized in that** the one or more light sources and the one or more light detectors are positioned in a single housing and the housing comprises an adhesive patch for attaching the housing to the subject's clothing.

11. Method for non-invasively measuring blood oxygen saturation in a subject's tissue, comprising the steps of: using at least one light source to emit a light signal into the subject's tissue, using at least one detector to detect the light signal reflected from the subject's tissue, using an electronic data processing unit to calculate the blood oxygen saturation based on the light signal measured by the one or more detector, using a motion sensor to detect a motion signal indicative of motion of the light source and/or the light detector, **characterized in that** the electronic data processing unit discards the light signal measured by the one or more detectors, if the motion signal detected by the motion sensor exceeds a predetermined threshold value.

12. A spectrophotometric device for measuring blood oxygen saturation in a subject's tissue comprising at least one light source for emitting a light signal into the subject's tissue, at least one detector for detecting the light signal reflected by the subject's tissue, an electronic data processing unit configured to calculate the blood oxygen saturation taking into account the light signal measured by the at least one detector, and a contact sensor for detecting a contact signal indicative of contact between the subject's skin and the light source and/or the detector.

13. The spectrophotometric device according to claim 12, **characterized in that** the electronic data processing unit is configured to discard the light signal measured by the at least one detector and/or to issue a warning to the user, if the contact signal indicates that at least one light source and/or at least one detector have lost contact to the subject's skin.

14. A method for non-invasively measuring blood oxygen saturation in a subject's tissue, comprising the steps of: using at least one light source to emit a light signal into the subject's tissue, using at least one detector to detect the light signal reflected from the subject's tissue, using an electronic data processing unit to calculate the blood oxygen saturation based on the light signal measured by the one or more detector, using a contact sensor for detecting a contact signal indicative of contact between the subject's skin and the at least one light source and/or the at least one detector, **characterized in that** the electronic data processing unit discards the light signal measured by the at least one detector and/or issues a warning to the user, if the contact signal indicates that at least one light source and/or at least one detector have lost contact to the subject's skin.

Europäisches
Patentamt

European
Patent Office

Office européen
des brevets

## EUROPEAN SEARCH REPORT

Application Number

EP 22 17 3473

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/383628 A1 (BORREMANS JONATHAN [BE] ET AL) 10 December 2020 (2020-12-10) <br> * abstract; figures 1-13 * <br> * paragraphs [0046] – [0116] * <br> ----- | 1-14 | INV. <br> A61B5/00 <br> A61B5/1455 <br> A61B5/113 |
| X | WO 2016/097271 A2 (KONINKL PHILIPS NV [NL]) 23 June 2016 (2016-06-23) <br> * abstract; figures 1-15 * <br> * page 10, line 26 – page 31, line 7 * <br> ----- | 1-14 | |
| X | US 2017/325698 A1 (ALLEC NICHOLAS PAUL JOSEPH [US] ET AL) 16 November 2017 (2017-11-16) <br> * abstract; figures 1-23 * <br> * paragraphs [0063] – [0150] * <br> ----- | 1-14 | |

TECHNICAL FIELDS SEARCHED (IPC)

A61B

The present search report has been drawn up for all claims

| Place of search | Date of completion of the search | Examiner |
|---|---|---|
| The Hague | 14 October 2022 | Carta, Riccardo |

EPO FORM 1503 03.82 (P04C01)

## ANNEX TO THE EUROPEAN SEARCH REPORT
## ON EUROPEAN PATENT APPLICATION NO.

EP 22 17 3473

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2022

| Patent document cited in search report | | Publication date | Patent family member(s) | | Publication date |
|---|---|---|---|---|---|
| US 2020383628 | A1 | 10-12-2020 | CN | 111683588 A | 18-09-2020 |
| | | | DE | 112019000207 T5 | 06-08-2020 |
| | | | US | 2020383628 A1 | 10-12-2020 |
| | | | WO | 2019141869 A1 | 25-07-2019 |
| WO 2016097271 | A2 | 23-06-2016 | BR | 112017012758 A2 | 26-12-2017 |
| | | | CN | 106999058 A | 01-08-2017 |
| | | | EP | 3232904 A1 | 25-10-2017 |
| | | | JP | 6684282 B2 | 22-04-2020 |
| | | | JP | 2018504171 A | 15-02-2018 |
| | | | RU | 2017125449 A | 23-01-2019 |
| | | | US | 2017347957 A1 | 07-12-2017 |
| | | | WO | 2016096391 A1 | 23-06-2016 |
| | | | WO | 2016097271 A2 | 23-06-2016 |
| US 2017325698 | A1 | 16-11-2017 | CN | 109152530 A | 04-01-2019 |
| | | | EP | 3454724 A1 | 20-03-2019 |
| | | | KR | 20180128492 A | 03-12-2018 |
| | | | US | 2017325698 A1 | 16-11-2017 |
| | | | WO | 2017197033 A1 | 16-11-2017 |

EPO FORM P0459

For more details about this annex : see Official Journal of the European Patent Office, No. 12/82

## REFERENCES CITED IN THE DESCRIPTION

**Patent documents cited in the description**

- EP 1259791 B1

- US 20120136225 A1

**Non-patent literature cited in the description**

- **TACHTSIDIS, ILIAS et al.** A Hybrid Multi-Distance Phase and Broadband Spatially Resolved Spectrometer and Algorithm for Resolving Absolute Concentrations of Chromophores in the Near-Infrared Light Spectrum. *Advances in Experimental Medicine and Biology,* 2010, vol. 662, 169-175